(19) Europäisches Patentamt
European Patent Office
Office européen des brevets

(11) **EP 3 779 016 B1**

(12) **EUROPEAN PATENT SPECIFICATION**

(45) Date of publication and mention
of the grant of the patent:
**19.04.2023 Bulletin 2023/16**

(21) Application number: **19775416.1**

(22) Date of filing: **08.03.2019**

(51) International Patent Classification (IPC):
**B32B 5/26** *(2006.01)*    **B32B 5/02** *(2006.01)*
**B32B 5/04** *(2006.01)*    **B32B 5/08** *(2006.01)*
**D04H 3/007** *(2012.01)*    **D04H 3/153** *(2012.01)*
**A41D 13/11** *(2006.01)*    **A61F 13/49** *(2006.01)*

(52) Cooperative Patent Classification (CPC):
**A61F 13/4902; B32B 3/26; B32B 5/022;**
**B32B 5/024; B32B 5/026; B32B 5/06; B32B 5/08;**
**B32B 5/26; B32B 7/12; B32B 27/12; B32B 27/18;**
**B32B 27/32; D04H 3/007; D04H 3/153;**
B32B 2250/02;                    (Cont.)

(86) International application number:
**PCT/JP2019/009429**

(87) International publication number:
**WO 2019/188134 (03.10.2019 Gazette 2019/40)**

(54) **NON-WOVEN FABRIC LAMINATE, STRETCHABLE NON-WOVEN FABRIC LAMINATE, AND TEXTILE PRODUCT**

VLIESSTOFFLAMINAT, DEHNBARES VLIESSTOFFLAMINAT, SOWIE TEXTILPRODUKT

STRATIFIÉ DE TISSU NON TISSÉ, STRATIFIÉ DE TISSU NON TISSÉ ÉTIRABLE, ET PRODUIT TEXTILE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.03.2018 JP 2018068200**

(43) Date of publication of application:
**17.02.2021 Bulletin 2021/07**

(73) Proprietor: **Mitsui Chemicals, Inc.**
**Minato-ku**
**Tokyo 105-7122 (JP)**

(72) Inventors:
• **TAKAKU, Shouichi**
 **Nagoya-shi, Aichi 457-8522 (JP)**
• **MOTOMURA, Shigeyuki**
 **Sodegaura-shi, Chiba 299-0265 (JP)**
• **SHIMADA, Koichi**
 **Sodegaura-shi, Chiba 299-0265 (JP)**
• **IIHAMA, Sho**
 **Sodegaura-shi, Chiba 299-0265 (JP)**

(74) Representative: **Hoffmann Eitle**
**Patent- und Rechtsanwälte PartmbB**
**Arabellastraße 30**
**81925 München (DE)**

(56) References cited:
**WO-A1-2007/138733    WO-A1-2016/143833**
**WO-A1-2016/143834    JP-A- 2011 514 938**

(52) Cooperative Patent Classification (CPC): (Cont.)
B32B 2250/03; B32B 2250/20; B32B 2262/0215;
B32B 2262/0253; B32B 2262/04; B32B 2262/10;
B32B 2262/14; B32B 2264/02; B32B 2270/00;
B32B 2274/00; B32B 2307/51; B32B 2307/516;
B32B 2307/518; B32B 2307/546; B32B 2307/718;
B32B 2307/72; B32B 2307/724; B32B 2307/728;
B32B 2437/00; B32B 2535/00; B32B 2553/00;
B32B 2555/00; B32B 2555/02

**Description**

Technical Field

The present disclosure relates to a multilayer nonwoven fabric, a stretchable multilayer nonwoven fabric, a fiber product, an absorbent article, and a sanitary mask.

Background Art

In recent years, nonwoven fabrics are widely used in various applications because of their excellent air breathability and flexibility. For that reason, the nonwoven fabrics require various characteristics in accordance with their applications, and improvements in their characteristics.

For example, excellent waterproofness and moisture permeability are required for nonwoven fabrics which are used for hygiene materials such as disposable diapers and sanitary napkins, and for backings for poultices, and the like. In addition, stretchability and bulkiness are also required depending on the sections to which the fabrics are used.

As a method of imparting stretchability to a nonwoven fabric, a method of using a thermoplastic elastomer as a raw material of a spunbond nonwoven fabric (see, for example, Japanese National-Phase Publication (JP-A) No. H07-503502), and a method of using a low crystalline polypropylene (see, for example, Japanese Patent Application Laid-Open (JP-A) Nos. 2009-62667 and 2009-79341), and the like have been proposed.

JP-A Nos. 2009-62667 and 2009-79341 propose that a highly crystalline polypropylene or a release agent is added to a low crystalline polypropylene in order to improve stickiness and the like of a spunbond nonwoven fabric. International Publication (WO-A) No. 2016/143833 discloses a laminated body of a nonwoven fabric including low crystalline polypropylene and a mixed-fiber spunbond nonwoven fabric including long fibers of a thermoplastic elastomer and long fibers of a thermoplastic resin.

WO 2007/138733 A1 relates to a non-woven fabric laminate comprising at least one meltblown non-woven fabric layer and a mixed-fiber spunbonded non-woven fabric layer laminated on the both sides of the meltblown non-woven fabric layer, wherein the mixed spunbonded non-woven fabric layer comprises a long fiber compose of a thermoplastic elastomer (A) and a long fiber composed of a thermoplastic resin (B) which is different from the thermoplastic elastomer (A). WO 2016/143833 A1 concerns nonwoven fabric laminates containing an elastic nonwoven fabric and a stretchable spunbonded nonwoven fabric.

WO 2009/111185 A2 is directed to bicomponent fibers made from ethylene/alpha-olefin interpolymers and to respective factrics.

SUMMARY OF INVENTION

Technical Problem

According to the methods described in JP-A Nos. 2009-62667 and 2009-79341, in order to prevent a spunbond nonwoven fabrics containing a low crystalline polypropylene from adhering to various rotary units in apparatuses during steps such as embossing or to any other regions which are brought into contact with the nonwoven fabrics during production of the spunbond nonwoven fabric using the low crystalline polypropylene, it is necessary to increase the amount of the highly crystalline polypropylene or release agent to be added to the low crystalline polypropylene. As a result, the resulting spunbond nonwoven fabric tends to have a greater residual strain and to be inferior in stretchability. According to the method described in IWO-A No. 2016/143833, stretchability is maintained by stacking a low crystalline polypropylene and an extensible spunbond nonwoven fabric, but a further improvement in stretchability is strongly demanded.

In applications such as hygiene materials (such as disposable diapers and sanitary napkins), and backings for poultices, elongation stress is required to be small so that the materials can be mounted with a weak force, and recovery stress is required to be large so as to prevent gap while being mounted. That is, in the above applications, it is required not only to decrease the value of stretch characteristics (ratio of elongation stress/recovery stress) but also to decrease the absolute value of the elongation stress and to increase the absolute value of the recovery stress.

Furthermore, in applications for sanitary materials such as disposable diapers and sanitary napkins, base fabrics of poultices, and the like, a recovery stress is required not to decrease, in other words, it is required to be excellent in stress maintainability, in a range of from a room temperature (23°C) to a body temperature (in a range of from 23°C to 40°C, for example). Being excellent in stress maintainability prevents sanitary materials such as disposable diapers and sanitary napkins from easily slipping off even in a case in which the materials attain a body temperature when being put on.

In view of the above-described problems, an object of the disclosure is to provide a multilayer nonwoven fabric excellent in a stretch property and excellent in stress maintainability, and a stretchable multilayer nonwoven fabric, a fiber product, an absorbent article, and a sanitary mask that use the multilayer nonwoven fabric.

Solution to Problem

Solutions to the above-described problems include the following aspects.

<1> A multilayer nonwoven fabric, including:

an elastic nonwoven fabric containing an α-olefin copolymer in which a ratio (E40/E23) between a storage elastic modulus E40 at 40°C and a storage elastic modulus E23 at 23°C is 37% or higher; and a mixed-fiber spunbond nonwoven fabric that is disposed on at least one side of the elastic nonwoven fabric, wherein the α-olefin copolymer includes a copolymer of ethylene and propylene, and wherein the α-olefin copolymer includes a copolymer of ethylene and propylene, and the mixed-fiber spunbond nonwoven fabric contains a long fiber of a thermoplastic elastomer (A) and a long fiber of a thermoplastic resin (B), which is different from the thermoplastic elastomer (A) and which is a polyolefin, in a ratio (A) : (B) of from 10 to 90% by mass : from 90 to 10% by mass, wherein (A) + (B) = 100% by mass.

<2> The multilayer nonwoven fabric according to <1>, wherein the storage elastic modulus E23 at 23°C of the α-olefin copolymer is 45 MPa or lower.
<3> The multilayer nonwoven fabric according to <1> or <3>, wherein the α-olefin copolymer has a tensile elastic modulus of 30 MPa or lower.
<4> The multilayer nonwoven fabric according to any one of <1> to <3>, wherein the α-olefin copolymer has a melting point of 145°C or lower.
<5> The multilayer nonwoven fabric according to any one of <1> to <4>, wherein the α-olefin copolymer has a fusion heat amount of 29 mJ/mg or smaller that is calculated from the highest endothermic peak measured by a differential scanning calorimeter (DSC).
<6> The multilayer nonwoven fabric according to any one of <1> to <5>, wherein the α-olefin copolymer has a glass transition temperature of -10°C or lower.
<7> The multilayer nonwoven fabric recited in any one of the above <1> to <6>, wherein a maximum point elongation of the long fiber of the thermoplastic resin (B), in a case of being formed into the spunbond nonwoven fabric, is 50% or more.
<8> The multilayer nonwoven fabric according to any one of <1> to <7>, wherein the thermoplastic elastomer (A) is a thermoplastic polyurethane elastomer.
<9> The multilayer nonwoven fabric according to <8>, wherein:

the thermoplastic polyurethane elastomer has a solidification starting temperature of 65°C or higher as measured by a DSC; and
a number of particles of the thermoplastic polyurethane elastomer insoluble in a dimethylacetamide solvent measured by a particle size distribution measuring apparatus equipped with an aperture of 100 μm based on a pore electrical resistance method, is 3,000,000/g or less.

<10> The multilayer nonwoven fabric according to 89> or <9>, wherein the thermoplastic polyurethane elastomer is a thermoplastic polyurethane elastomer satisfying the following relational expression (I):

$$a/(a + b) \le 0.8 \qquad (I)$$

wherein:

a represents a total amount of fusion heat calculated from an endothermic peak in a range of 90°C to 140°C, as measured by DSC; and
b represents a total amount of fusion heat calculated from an endothermic peak in a range from higher than 140°C to 220°C or lower, as measured by DSC.

<11> The multilayer nonwoven fabric according to any one of <1> to <9>, wherein the thermoplastic resin (B) is a propylene polymer.
<12> The multilayer nonwoven fabric according to any one of <1> to <11>, wherein the thermoplastic resin (B) includes 99 to 80% by mass of a propylene polymer and 1 to 20% by mass of a high-density polyethylene.

<13> A stretchable multilayer nonwoven fabric obtained by drawing the multilayer nonwoven fabric according to any one of <1> to <12>.

<14> A fiber product including the multilayer nonwoven fabric according to any one of <1> to <12> or the stretchable multilayer nonwoven fabric according to <13>.

<15> An absorbent article including the multilayer nonwoven fabric according to any one of <1> to <12> or the stretchable multilayer nonwoven fabric according to <13>.

<16> A sanitary mask including the multilayer nonwoven fabric according to any one of <1> to <12> or the stretchable multilayer nonwoven fabric according to <13>.

Advantageous Effects of Invention

According to the disclosure, a multilayer nonwoven fabric excellent in a stretch property and excellent in stress maintainability, and a stretchable multilayer nonwoven fabric, a fiber product, an absorbent article, and a sanitary mask that use the multilayer nonwoven fabric are provided.

BRIEF DESCRIPTION OF DRAWINGS

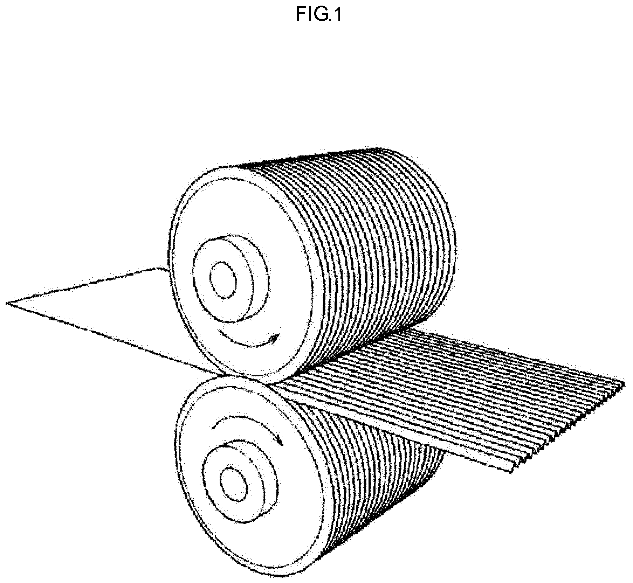

Fig. 1 is a schematic diagram of a gear drawing device.

DESCRIPTION OF EMBODIMENTS

Hereinafter, embodiments for carrying out the present invention will be described in detail. However, the invention is not limited to the following embodiments. In the following embodiments, constituent elements (including element steps and the like) are not indispensable unless otherwise expressly provided or except the case in which the constituent elements are apparently indispensable in principle. The same applies to numerical values and ranges of the constituent elements, and the invention is not limited thereby.

In the present specification, the term "step" includes not only a separate step but also a step that cannot be clearly distinguished from other steps as long as the object of the step is achieved. In the present specification, the notation "to" expressing a numerical range indicates a range including the numerical values before and after "to", as the minimum value and the maximum value, respectively. In the present specification, in a case in which plural kinds of substances corresponding to components exist in the composition, the content of each of the components in the composition refers to the total amount of the plural substances in the composition unless otherwise specified.

<Multilayer nonwoven fabric>

A multilayer nonwoven fabric of the disclosure includes an elastic nonwoven fabric including an $\alpha$-olefin copolymer in which a ratio (E40/E23) between a storage elastic modulus E40 at 40°C and a storage elastic modulus E23 at 23°C is 37% or higher, and a mixed-fiber spunbond nonwoven fabric that is disposed on at least one side of the elastic nonwoven fabric, wherein the $\alpha$-olefin copolymer includes a copolymer of ethylene and propylene, and the mixed-fiber spunbond nonwoven fabric contains a long fiber of a thermoplastic elastomer (A) and a long fiber of a thermoplastic resin (B), which is different from the thermoplastic elastomer (A) and which is a polyolefin, in a ratio (A) : (B) of from 10 to 90% by mass to 10% by mass, in which (A) + (B) = 100% by mass (hereinafter also simply referred to as a "mixed-fiber spunbond nonwoven fabric"). The multilayer nonwoven fabric may be configured to include other layers.

The multilayer nonwoven fabric according to the disclosure includes an $\alpha$-olefin copolymer as the elastic nonwoven fabric. Thus, it is considered that there can be obtained a multilayer nonwoven fabric that is more excellent in a stretch property and more excellent in stress maintainability than a multilayer nonwoven fabric that uses an elastic nonwoven fabric not including an $\alpha$-olefin copolymer, such as an elastic nonwoven fabric formed of a polypropylene homopolymer, for example.

The $\alpha$-olefin copolymer has a storage elastic modulus E40 at 40°C and a storage elastic modulus E23 at 23°C that are in a ratio (E40/E23) of 37% or higher. Thus, the elastic nonwoven fabric is easily prevented from being reduced in its elasticity even under a temperature changing environment (from 40°C to 23°C, for example). For this reason, it is considered that a multilayer nonwoven fabric having excellent stress maintainability can be obtained.

Since the mixed-fiber spunbond nonwoven fabric of the disclosure is disposed on at least one side of the elastic nonwoven fabric in the multilayer nonwoven fabric of the invention, the adhesion of the multilayer nonwoven fabric to members such as various rotating devices in an apparatus used in an embossing step and the like can be prevented, which provides excellent moldability. Since the spunbond nonwoven fabric disposed on at least one side of the elastic nonwoven fabric includes a specific mixed fiber, the multilayer nonwoven fabric is less sticky and excellent in stretchability.

This allows the elastic nonwoven fabric to easily keep its stretchability attributed to excellent elasticity.

The multilayer nonwoven fabric of the disclosure preferably has a structure in which at least a mixed-fiber spunbond nonwoven fabric is disposed on a surface brought into contact with rotating equipment attached to a nonwoven fabric producing apparatus This can avoid troubles in manufacture due to winding around the rotary device or the like. More preferably, the multilayer nonwoven fabric of the disclosure has a structure in which a mixed-fiber spunbond nonwoven fabric is disposed on each of both sides of an elastic nonwoven fabric. Those configurations make it possible to stably obtain a multilayer nonwoven fabric excellent in a stretch property and excellent in stress maintainability.

The multilayer nonwoven fabric of the disclosure usually has a basis weight of 360 g/m$^2$ or less, preferably 240 g/m$^2$ or less, more preferably 150 g/m$^2$ or less, and further preferably from 120 g/m$^2$ to 15 g/m$^2$. The basis weight can be measured by a method used in Examples described later.

The compositional ratio (basis weight ratio) of the elastic nonwoven fabric to the mixed-fiber spunbond nonwoven fabric may be appropriately determined according to various applications. Usually, the basis weight ratio (the elastic nonwoven fabric : mixed-fiber spunbond nonwoven fabric) is in the range of 10 : 90 to 90 : 10, preferably in the range of 20 : 80 to 80 : 20, and more preferably in the range of 20 : 80 to 50 : 50. In a case in which two or more elastic nonwoven fabrics (or mixed-fiber spunbond nonwoven fabrics) exist, the basis weight of the elastic nonwoven fabrics (or mixed-fiber spunbond nonwoven fabrics) is the total of the basis weights of the two or more elastic nonwoven fabrics.

The multilayer nonwoven fabric of the invention usually has residual strain of 19.5% or less in at least one direction, and preferably 19% or less. In a case in which the multilayer nonwoven fabric has residual strain of 19% or less in at least one direction, the multilayer nonwoven fabric has good stretchability. The residual strain can be measured by a method used in Examples described later.

The multilayer nonwoven fabric of the disclosure usually has a maximum load elongation of 50% or more in at least one direction, and preferably 100% or more. The maximum load elongation can be measured by a method used in Examples described later.

In a case in which the multilayer nonwoven fabric of the invention has a basis weight of 65 g/m$^2$, the multilayer nonwoven fabric has stress at 50% extending of preferably 1.10 N/50 mm or less, more preferably 1.05 N/50 mm or less, and further preferably 1.00 N/50 mm or less. Stress at 50% recovering is preferably 0.30 N/50 mm or more, more preferably 0.32 N/50 mm or more, and further preferably 0.35 N/50 mm or more. The stress at 50% extending and the stress at 50% recovering differ from stretch characteristics (stress at 50% extending/stress at 50% recovering), and tend to change depending on the basis weight of the multilayer nonwoven fabric, and tend to be increased as the basis weight is increased.

The multilayer nonwoven fabric of the disclosure has stretch characteristics (stress at 50% extending/stress at 50% recovering) of preferably 3.0 or less, more preferably 2.7 or less, and further preferably 2.6 or less. As the value of the stretch characteristics is smaller, the stretch characteristics are more excellent. The stretch characteristics can be measured by a method used in Examples described later.

<Elastic nonwoven fabric>

The elastic nonwoven fabric forming the multilayer nonwoven fabric of the disclosure includes an α-olefin copolymer, which includes a copolymer of ethylene and propylene, and in which a ratio (E40/E23) between a later-described storage elastic modulus E40 at 40°C and a storage elastic modulus E23 at 23°C is 37% or higher. From the viewpoint of effectively attaining an object of the invention, the percentage of the α-olefin copolymer in which a ratio (E40/E23) between a storage elastic modulus E40 at 40°C and a storage elastic modulus E23 at 23°C is 37% or higher in the elastic nonwoven fabric, is preferably 60% by mass or more, more preferably 70% by mass or more, still more preferably 80% by mass or more, and particularly preferably 100% by mass. Examples of the α-olefin copolymer in which a ratio (E40/E23) between a storage elastic modulus E40 at 40°C and a storage elastic modulus E23 at 23°C is 37% or higher include VISTAMAXX (trade name, manufactured by Exxon Mobile Chemical Ltd.).

In the disclosure, the elastic nonwoven fabric refers to a nonwoven fabric having an elastic recovering property in a case in which stress is released after drawing.

The elastic nonwoven fabric can be produced by various known methods. Specific examples thereof include a spunbond method, a melt blow method, and a flash spinning method. Among the elastic nonwoven fabrics, a spunbond nonwoven fabric obtained by the spunbond method or a meltblown nonwoven fabric obtained by the melt blow method is preferable.

The elastic nonwoven fabric usually has a basis weight of 120 g/m$^2$ or less, preferably 80 g/m$^2$ or less, more preferably 50 g/m$^2$ or less, and further preferably from 40 g/m$^2$ to 2 g/m$^2$.

The fiber constituting the elastic nonwoven fabric usually has a fiber diameter of 50 μm or less, preferably 40 μm or less, and more preferably 35 μm or less.

[α-Olefin copolymer in which ratio (E40/E23) between storage elastic modulus E40 at 40°C and storage elastic modulus E23 at 23°C is 37% or higher]

The elastic nonwoven fabric includes an α-olefin copolymer in which a ratio (E40/E23) between a storage elastic modulus E40 at 40°C and a storage elastic modulus E23 at 23°C is 37% or higher (hereinafter also referred to as an "α-olefin copolymer").

The α-olefin copolymer represents a copolymer in which two or more kinds of copolymeric components each having an α-olefin skeleton are copolymerized.

Examples of a copolymeric component having an α-olefin skeleton include α-olefins such as ethylene, propylene, 1-butene, 1-pentene, 3-methyl-1-butene, 4-methyl-1-pentene, 1-hexene, 1-octene, 1-decene, 1-dodecene, 1-tetradecene, 1-hexadecene, 1-octadecene, and 1-eicocene.

In the present invention, the α-olefin copolymer includes a copolymer of ethylene (hereinafter also referred to as "C2") and propylene (hereinafter also referred to as "C3") among the above-described examples, from the viewpoint of allowing the multilayer nonwoven fabric to have a lower stress and more excellent stretchability.

A content per component derived from ethylene in a copolymer of ethylene and propylene (hereinafter also simply referred to as an "ethylene content") is preferably from 1% by mass to 50% by mass, more preferably from 5% by mass to 25% by mass, and still more preferably from 10% by mass to 20% by mass.

The α-olefin copolymer may be any of an alternating copolymer, a graft copolymer, a block copolymer, and a random copolymer.

The ratio (E40/E23) between a storage elastic modulus E40 at 40°C and a storage elastic modulus E23 at 23°C in the α-olefin copolymer is 37% or higher from the viewpoint of obtaining a multilayer nonwoven fabric excellent in stress maintainability. The higher the ratio (E40/E23) is, the more preferable it is. The ratio is more preferably 40% or higher, still more preferably 45% or higher, and particularly preferably 50% or higher.

Examples of a method of setting the ratio (E40/E23) between storage elastic moduli in the α-olefin copolymer to the above-described specific range include a method in which a copolymer of ethylene and propylene is used as the α-olefin copolymer, and the like.

From the viewpoint of making the multilayer nonwoven fabric more excellent in stretchability, the storage elastic modulus E23 at 23°C of the α-olefin copolymer is preferably 45 MPa or lower, is more preferably 35 MPa or lower, and is still more preferably 25 MPa or lower.

E40/E23 is preferably 37% or higher, is more preferably 40% or higher, is still more preferably 45% or higher, and is particularly preferably 50% or higher.

As each storage elastic modulus of the α-olefin copolymer, a value measured using the following apparatus and under the following conditions is used.

Temperature: 23°C or 40°C
Apparatus: RSA-III (manufactured by TA Instruments Inc.)
Deformation mode: tensile mode
Temperature range: from -20°C to 120°C
Rate of temperature increase: 2°C/min.
Deformation frequency: 10 Hz
Initial strain: 0.1%
Measured temperature sensation: 0.3°C
Environment: $N_2$

The melting point of the α-olefin copolymer used in the elastic nonwoven fabric of the disclosure is defined as a peak top of a peak observed on the highest-temperature side of a melting endothermic curve that is obtained by temperature increase of a sample at 10°C/min. after the sample is held in a nitrogen atmosphere at -100°C for five minutes, using a differential scanning calorimeter (DSC). More specifically, the melting point of the α-olefin copolymer can be obtained as a peak top of a peak observed on the highest-temperature side of a melting endothermic curve that is obtained by temperature increase of five milligrams of sample at 10°C/min. after the sample is held in a nitrogen atmosphere at -100°C for five minutes, using a differential scanning calorimeter (manufactured by Perkin Elmer Incorporation, DSC-7).

The melting point of the α-olefin copolymer used for the elastic nonwoven fabric of the disclosure is preferably 145°C or lower, more preferably 130°C or lower, and still more preferably 115°C or lower.

The amount of fusion heat of the α-olefin copolymer used for the elastic nonwoven fabric of the disclosure is defined as a fusion heat amount at the highest endothermic peak in a melting endothermic curve that is obtained by temperature increase of a sample at 10°C/min. after the sample is held in a nitrogen atmosphere at -100°C for five minutes, using a differential scanning calorimeter (DSC). More specifically, the fusion heat amount of the α-olefin copolymer can be obtained from the highest endothermic peak in a melting endothermic curve that is obtained by temperature increase of five milligrams of sample at 10°C/min. after the sample is held in a nitrogen atmosphere at -100°C for five minutes, using a differential scanning calorimeter (manufactured by Perkin Elmer Incorporation, DSC-7).

The fusion heat amount of the α-olefin copolymer used for the elastic nonwoven fabric of the disclosure is preferably 29 mJ/mg or smaller, more preferably 25 mJ/mg or smaller, and still more preferably 20 mJ/mg or smaller.

The glass transition temperature of the α-olefin copolymer used for the elastic nonwoven fabric of the disclosure is defined as a temperature at which there is a made a shift of a base line of a melting endothermic curve that is obtained by temperature increase of a sample at 10°C/min. after the sample is held in a nitrogen atmosphere at -100°C for five minutes, using a differential scanning calorimeter (DSC). More specifically, an intersection point of a base line of a melting endothermic curve that is obtained by temperature increase of five milligrams of sample at 10°C/min. after the sample is held in a nitrogen atmosphere at -100°C for five minutes, using a differential scanning calorimeter (manufactured by Perkin Elmer Incorporation, DSC-7), and a tangent at an inflection point, can be obtained as the glass transition temperature.

The glass transition temperature of the α-olefin copolymer used for the elastic nonwoven fabric of the disclosure is preferably -10°C or lower, more preferably -20°C or lower, and still more preferably -25°C or lower.

The density (ASTM D1505) of the α-olefin copolymer used for the elastic nonwoven fabric of the disclosure is preferably in a range of from 0.850 g/cm$^3$ to 0.950 g/cm$^3$, more preferably in a range of from 0.855 g/cm$^3$ to 0.900 g/cm$^3$, and still more preferably in a range of from 0.860 g/cm$^3$ to 0.895 g/cm$^3$.

The density of the α-olefin copolymer is a value obtained by measurement in accordance with a density gradient method complying with JIS K7112 (1999).

The tensile elastic modulus of the α-olefin copolymer used for the elastic nonwoven fabric of the disclosure is preferably 30 MPa or lower, more preferably 20 MPa or lower, and still more preferably 15 MPa or lower, from the viewpoint of making the multilayer nonwoven fabric more excellent in stretchability. The tensile elastic modulus is a value obtained by measurement using a method complying with JIS K7161 (2011).

The molecular-weight distribution (Mw/Mn) of the α-olefin copolymer used for the elastic nonwoven fabric of the disclosure is preferably in a range of from 1.5 to 5.0. Mw/Mn is more preferably in a range of from 1.5 to 4.5 in that a fiber having excellent spinnability and significantly-excellent strength can be obtained.

The mass average molecular weight (Mw) and the number average molecular weight (Mn) of the α-olefin copolymer used for the elastic nonwoven fabric of the disclosure are values calculated by gel permeation chromatography (GPC) and are values measured under the following conditions. The mass average molecular weight (Mw) is a mass average molecular weight measured by polystyrene standards, and the molecular-weight distribution (Mw/Mn) is a value calculated from the number average molecular weight (Mn) measured in the same manner and the mass average molecular weight (Mw).

<GPC Measurement conditions>

Column: TOSO GMHHR-H(S) HT
Detector: liquid-chromatogram RI detector, WATERS 150C
Solvent: 1, 2, 4-trichlorobenzene
Measurement temperature: 145°C
Flow velocity: 1.0 ml/min.
Sample concentration: 2.2 mg/ml
Dose: 160 μl
Calibration curve: Universal Calibration
Analysis program: HT-GPC (Ver. 1.0)

The melt flow rate (MFR) of the α-olefin copolymer used for the elastic nonwoven fabric of the disclosure is not limited to a particular rate. For example, the melt flow rate is preferably from 1 g/10 min. to 100 g/10 min., more preferably from 10 g/10 min. to 80 g/10 min., and still more preferably from 15 g/10 min. to 70 g/10 min.

The melt flow rate of the α-olefin copolymer used for the elastic nonwoven fabric of the disclosure is measured under the conditions of ASTM D-1238, 230°C, and a load of 2160 g.

The α-olefin copolymer used for the elastic nonwoven fabric of the disclosure may be a synthetic product, and may be a commercially-available product.

In a case in which the α-olefin copolymer is a synthetic product, it is possible to prepare the α-olefin copolymer by polymerizing or copolymerizing a monomer in the presence of any of conventionally-known catalysts such as a Ziegler-Natta catalyst and a metallocene-based catalyst, using any of conventionally-known polymerization methods such as a vapor phase method, a bulk method, a slurry method, and a solution method.

Examples of a commercially-available product for the α-olefin copolymer include VISTAMAXX series (manufactured by Exxon Mobile Chemical Ltd.) and the like.

Composition of the $\alpha$-olefin copolymer can be achieved by a conventionally-known method (IR analysis, NMR analysis, microanalysis, or the like, for example).

The percentage of the $\alpha$-olefin copolymer to the total amount of the elastic nonwoven fabric is preferably from 90% by mass to 100% by mass and more preferably from 98% by mass to 100% by mass.

The $\alpha$-olefin copolymer used for the elastic nonwoven fabric of the disclosure may include various known additives such as an antioxidant, a heat-resistance stabilizer, a weather-resistant stabilizer, an antistatic agent, a slip agent, an antifogging agent, a lubricant, a dye, a pigment, natural oil, synthetic oil, and wax, as optional components, as long as the object of the invention is not impaired.

<Mixed-fiber spunbond nonwoven fabric>

The mixed-fiber spunbond nonwoven fabric forming the multilayer nonwoven fabric of the disclosure includes a long fiber of a thermoplastic elastomer (A) and a long fiber of a thermoplastic resin (B), which is different from the thermoplastic elastomer (A) and which is a polyolefin, in a ratio (A) : (B) of from 10 to 90% by mass : from 90 to 10% by mass, wherein (A) + (B) = 100% by mass.

From the viewpoint of stretchability and flexibility, the proportion of the long fibers of the thermoplastic elastomer (A) in the mixed-fiber spunbond nonwoven fabric is preferably 20% by mass or more, and more preferably 30% by mass or more. From the viewpoint of processability (sticky resistance), the proportion of the long fibers of the thermoplastic elastomer (A) in the mixed-fiber spunbond nonwoven fabric is preferably 70% by mass or less, and more preferably 60% by mass or less.

The fiber diameters (average value) of the long fibers of each of the thermoplastic elastomer (A) and the thermoplastic resin (B) that form the mixed-fiber spunbond nonwoven fabric are usually 50 $\mu$m or less, preferably 40 $\mu$m or less, and more preferably 35 $\mu$m or less. The fiber diameter of the long fibers of the thermoplastic elastomer (A) and the fiber diameter of the long fibers of the thermoplastic resin (B) may be the same or different.

In applications for sanitary materials such as diapers, from the viewpoint of flexibility and air breathability, the mixed-fiber spunbond nonwoven fabric generally has a basis weight of 120 g/m$^2$ or less, preferably 80 g/m$^2$ or less, more preferably 50 g/m$^2$ or less, and further preferably from 40 g/m$^2$ to 15 g/m$^2$ in the total multilayer.

[Thermoplastic elastomer (A)]

As the thermoplastic elastomer (A), various known thermoplastic elastomers can be used. One kind of the thermoplastic elastomers may be used alone, or two or more kinds of the thermoplastic elastomers may be used in combination.

Examples of the thermoplastic elastomer (A) include a styrene elastomer, a polyester elastomer, a polyamide elastomer, a thermoplastic polyurethane elastomer, a polyolefin elastomer, a vinyl chloride elastomer, and a fluorine elastomer.

The styrene elastomer is an elastomer typified by a polystyrene-polybutadiene-polystyrene block copolymer (SBS), a polystyrene-polyisoprene-polystyrene block copolymer (SIS), and a polystyrene-polyethylene butylene-polystyrene block copolymer (SEBS), and a polystyrene-polyethylene propylene-polystyrene block copolymer (SEPS) which are hydrogenation products thereof. The styrene elastomer is a block copolymer composed of at least one polymer block composed of an aromatic vinyl compound such as styrene and at least one conjugated diene compound such as butadiene or isoprene, or a hydrogenated product thereof. Examples of the styrene elastomer include KRATON polymer (trade name, manufactured by Shell Chemical Co.), SEPTON (trade name, manufactured by Kuraray Co., Ltd.), TLTFTEC (trade name, manufactured by Asahi Chemical Industry Co., Ltd.), and LEOSTOMER (trade name, manufactured by RIKEN TECHNOS CORPORATION).

The polyester elastomer is an elastomer typified by a block copolymer composed of a highly crystalline aromatic polyester and a noncrystalline aliphatic polyether. The polyester elastomer is sold under the trade names of, for example, HYTREL (trade name, manufactured by EI du Pont de Nemours and Company) and PELPRENE (trade name, manufactured by Toyobo Co., Ltd.).

The polyamide elastomer is an elastomer typified by a block copolymer composed of crystalline polyamide having a high melting point and a noncrystalline polyether or polyester having a low glass transition temperature (Tg). The polyamide elastomer is sold under the trade name of PEBAX (trade name, Atofina Japan Ltd.), for example.

The thermoplastic polyurethane elastomer is an elastomer typified by a block copolymer in which a hard segment is composed of polyurethane and a soft segment is composed of a polycarbonate polyol, an ether polyol, a caprolactone polyester, or an adipate polyester and the like.

The polyolefin elastomer is an elastomer containing a simple body such as a noncrystalline or low crystalline ethylene·$\alpha$-olefin random copolymer, a propylene·$\alpha$-olefin random copolymer, or a propylene·ethylene-$\alpha$-olefin random copolymer; or an elastomer as a mixture of the noncrystalline or low crystalline random copolymer with a propylene homopolymer; or an elastomer as a mixture of the noncrystalline or low crystalline random copolymer with crystalline polyolefin such as a copolymer of propylene and a small amount of an α-olefin, a high density polyethylene, and a medium density polyethylene. The polyolefin elastomer is sold under the trade names of, for example, TAFMER (trade name, manufactured by Mitsui Chemicals, Inc.), Engage (trade name, manufactured by DuPont Dow Elastomers) which is an ethylene-octene copolymer, CATALLOY containing a crystalline olefin copolymer (trade name, manufactured by Montel Co., Ltd.), and Vistamaxx (trade name, manufactured by Exxon Mobil Chemical Co., Ltd.), and the like.

The vinyl chloride elastomer is sold under the trade names such as Leonyl (trade name, manufactured by Riken Technos Co., Ltd.), and POSMYL (trade name, manufactured by Shin-Etsu Polymer Co., Ltd.).

Among these thermoplastic elastomers, the thermoplastic polyurethane elastomer and the polyolefin elastomer are preferable, and the thermoplastic polyurethane elastomer is more preferable from the viewpoint of stretchability and processability.

(Thermoplastic polyurethane elastomer)

Among the thermoplastic polyurethane elastomers, thermoplastic polyurethane elastomers having a solidification starting temperature of 65°C or higher, preferably 75°C or higher, and most preferably 85°C or higher are preferable. The solidification starting temperature is preferably 195°C or lower. Here, the solidification starting temperature is a value measured using a differential scanning calorimeter (DSC), and is the starting temperature of an exothermic peak derived from the solidification of the thermoplastic polyurethane elastomer occurring in a case in which the temperature of the thermoplastic polyurethane elastomer is decreased at a rate of 10°C/min after the temperature of the thermoplastic polyurethane elastomer is increased at 10°C/min to 230°C and kept at 230°C for 5 min. In a case in which the solidification starting temperature is 65°C or higher, it is possible to suppress defective molding such as fusion of fibers, filament breakage, massed resin in obtaining a mixed-fiber spunbond nonwoven fabric, and also it is possible to prevent the molded mixed-fiber spunbond nonwoven fabrics from being wound around the emboss roller in thermal emboss processing. The obtained mixed-fiber spunbond nonwoven fabrics have less stickiness, and are suitably used as materials that come in contact with skin such as clothes, hygiene materials, and materials of sporting goods. On the other hand, by setting the solidification starting temperature to 195°C or lower, the molding processability can be improved. The solidification starting temperature of the molded fiber tends to be higher than the solidification starting temperature of the thermoplastic polyurethane elastomer used for the fiber.

In order to adjust the solidification starting temperature of the thermoplastic polyurethane elastomer to 65°C or higher, it is necessary to select a polyol, an isocyanate compound, and a chain extender each having an optimum chemical structure, which are used as raw materials for the thermoplastic polyurethane elastomer, and to adjust a hard segment amount. The term "hard segment amount" as used herein is a mass percent value (% by mass) determined by dividing the total mass of the isocyanate compound and the chain extender used is in the production of the thermoplastic polyurethane elastomer by the total amount of the polyol, the isocyanate compound, and the chain extender, and multiplying the resulting value by 100. The hard segment amount is preferably from 20% by mass to 60% by mass, more preferably from 22% by mass to 50% by mass, and further preferably from 25% by mass to 48% by mass.

The thermoplastic polyurethane elastomer has the number of particles as components insoluble in a polar solvent of preferably 3,000,000 particles (3,000,000 particles/g) or less, more preferably 2,500,000 particles/g or less, and further preferably 2,000,000 particles/g or less with respect to 1 g of the thermoplastic polyurethane elastomer. Here, the components insoluble in the polar solvent in the thermoplastic polyurethane elastomer are mainly agglomerates such as fish eye and gel occurring during the production of the thermoplastic polyurethane elastomer. Examples of components causing the occurrence of the components insoluble in the polar solvent include components derived from hard segment agglomerates of the thermoplastic polyurethane elastomer, components obtained by crosslinking the hard segment and/or the soft segment with an allophanate bond or a biuret bond and the like, and components produced by a chemical reaction between raw materials.

The number of particles as the components insoluble in the polar solvent is determined by dissolving the thermoplastic polyurethane elastomer in a dimethylacetamide solvent and measuring the insoluble components by means of a particle size distribution measuring apparatus equipped with a 100 μm aperture utilizing a pore electrical resistance method. Using the apparatus equipped with a 100 μm aperture, the number of particles of from 2 to 60 μm can be measured in terms of uncrosslinked polystyrene.

By setting the number of particles as the components insoluble in the polar solvent to 3,000,000 particles/g or less, problems such as an increase in a distribution of a fiber diameter, and filament breakage in spinning can be further suppressed in the solidification starting temperature range of the thermoplastic polyurethane elastomer. The thermoplastic polyurethane elastomer having a low content of the components insoluble in the polar solvent can be obtained by conducting a polymerization reaction of a polyol, an isocyanate compound, and a chain extender, followed by filtration.

From the viewpoint of suppressing mingling of bubbles into a strand or occurrence of filament breakage in the molding of nonwoven fabrics using a large-size spunbonding molding machine, the thermoplastic polyurethane elastomer has a water content of preferably 350 ppm or less, more preferably 300 ppm or less, and further preferably 150 ppm or less.

From the viewpoint of stretchability, in the thermoplastic polyurethane elastomer, a total amount (a) of fusion heat calculated from an endothermic peak at a peak temperature in a range of 90°C to 140°C as measured by a differential scanning calorimeter (DSC) and a total amount (b) of fusion heat calculated from an endothermic peak at a peak temperature of from higher than 140°C to 220°C or lower preferably satisfy the following relational expression (I), more preferably satisfy the following relational expression (II), and further preferably satisfy the following relational expression (III).

$$a/(a+b) \leq 0.8 \qquad (I)$$

$$a/(a+b) \leq 0.7 \qquad (II)$$

$$a/(a+b) \leq 0.55 \qquad (III)$$

Here, "a/(a + b)" means a ratio (unit: %) of the fusion heat amount of the hard domain of the thermoplastic polyurethane elastomer. In a case in which the ratio of the fusion heat amount of the hard domain of the thermoplastic polyurethane elastomer is 80% or less, the strength and stretchability of the fiber, particularly the fiber and nonwoven fabric in the mixed-fiber spunbond nonwoven fabric are improved. In the disclosure, the lower limit of the ratio of the fusion heat amount of the hard domain of the thermoplastic polyurethane elastomer is preferably about 0.1%.

The thermoplastic polyurethane elastomer preferably has a melt viscosity of 100 Pa s to 3000 Pa·s under conditions of a temperature of 200°C and a shear rate of 100 sec$^{-1}$, more preferably from 200 Pa·s to 2000 Pa·s, and further preferably from 1000 Pa·s to 1500 Pa·s. Herein, the melt viscosity is a value measured by a capirograph (manufactured by Toyo Seiki Co., Ltd., nozzle length: 30 mm, diameter: 1 mm).

The thermoplastic polyurethane elastomer having such characteristics can be obtained by, for example, the production method described in JP-A No. 2004-244791.

The mixed-fiber spunbond nonwoven fabric molded by using the thermoplastic polyurethane elastomer is excellent in tactile sensation and therefore can be preferably used for applications that come in contact with skin such as a sanitary material. The thermoplastic polyurethane elastomer hardly causes clogging of a filter that is mounted in an extruder in order to filter impurities and the like, and reduces the frequency of adjustment and maintenance for equipment, which is industrially preferable.

(Polyolefin elastomer)

Among the polyolefin elastomers, noncrystalline or low crystalline polyolefin elastomers are preferable, and an ethylene·$\alpha$-olefin copolymer which is a copolymer of noncrystalline or low crystalline ethylene and one or more $\alpha$-olefins having 3 to 20 carbon atoms such as propylene, 1-butene, 1-pentene, 1-hexene, 4-methyl-1-pentene, 1-heptene, 1-octene, and 1-decene, and a propylene·$\alpha$-olefin copolymer which is a copolymer of noncrystalline or low crystalline propylene and one or more $\alpha$-olefins having 2 to 20 carbon atoms (excluding 3 carbon atoms) such as ethylene, 1-butene, 1-pentene, 1-hexene, 4-methyl-1-pentene, 1-heptene, 1-octene, and 1-decene are more preferable. The noncrystalline or low crystalline polyolefin elastomer is, for example, a polyolefin elastomer having a degree of crystallinity of 20% or less (including 0%) as measured by X-ray diffraction.

Specific examples of the noncrystalline or low crystalline ethylene·$\alpha$-olefin copolymer include an ethylene·propylene random copolymer, and an ethylene·1-butene random copolymer. The melt flow rate (MFR) of the ethylene·$\alpha$-olefin copolymer is not particularly limited as long as the ethylene·$\alpha$-olefin copolymer has spinnability, but MFR (ASTM D1238 190°C, load: 2160 g) is usually in the range of from 1 g/10 min to 1000 g/10 min, preferably from 5 g/10 min to 500 g/10 min, and more preferably from 10 g/10 min to 100 g/10 min.

Specific examples of the noncrystalline or low crystalline propylene·$\alpha$-olefin copolymer include a propylene·ethylene random copolymer, a propylene·ethylene·1-butene random copolymer, and a propylene·1-butene random copolymer. MFR of the propylene·$\alpha$-olefin copolymer is not particularly limited as long as the propylene·$\alpha$-olefin copolymer has spinnability, but MFR (ASTM D1238 230°C, load: 2160 g) is usually from 1 g/10 min to 1000 g/10 min, preferably from 5 g/10 min to 500 g/10 min, and more preferably from 10 g/10 min to 100 g/10 min.

The polyolefin elastomer may be a single body of a copolymer of noncrystalline or low crystalline polymer, and may also be a composition obtained by mixing a propylene homopolymer, a copolymer of propylene and a small amount of $\alpha$-olefin, or crystalline polyolefin such as a high density polyethylene or a medium density polyethylene in an amount of about 1 to about 40% by mass with the noncrystalline or low crystalline polymer.

A composition that is particularly preferable as the polyolefin elastomer is an elastomer composition containing a polypropylene resin composition containing 1 to 40% by mass of isotactic polypropylene (i) and 60 to 99% by mass of a propylene·ethylene·$\alpha$-olefin copolymer (ii) (a copolymer of 45 to 89 mol% of propylene, 10 to 25 mol% of ethylene, and $\alpha$-olefin having 4 to 20 carbon atoms, the amount of copolymerized $\alpha$-olefin having 4 to 20 carbon atoms does not exceed 30 mol%).

[Thermoplastic resin (B)]

In the present invention, the thermoplastic resin (B) is a polyolefin and different from the thermoplastic elastomer (A). The thermoplastic resins may be used alone, or in combination of two or more.

The thermoplastic resin (B) is a resinous polymer different from the thermoplastic elastomer (A), and is usually a crystalline polymer having a melting point (Tm) of 100°C or higher, or a noncrystalline polymer having a glass transition temperature of 100°C or higher. As the thermoplastic resin (B), the crystalline thermoplastic resin is preferable.

Among the thermoplastic resins (B), it is preferable to use a thermoplastic resin (extensible thermoplastic resin) having a maximum point elongation of 50% or more, preferably 70% or more, and more preferably 100% or more and having quite low elastic recoverability in a case of a spunbond nonwoven fabric obtained by a known production method. A multilayer nonwoven fabric produced by using a mixed-fiber spunbond nonwoven fabric obtained by mixing long fibers of such a thermoplastic resin (B) with long fibers of the thermoplastic elastomer (A) can exhibit bulkiness due to drawing, and have improved tactile feeling and an extension stop function. The upper limit of the maximum point elongation of the spunbond nonwoven fabric made of the thermoplastic resin (B) is not necessarily limited, but it is usually 300% or less.

Specific examples of the polyolefins used as the thermoplastic resin (B) incude homopolymers or copolymers of $\alpha$-olefins including ethylene, propylene, 1-butene, 1-hexene, 4-methyl-1-pentene, and 1-octene, including polyolefins such as high pressure low density polyethylene, linear low density polyethylene (so-called LLDPE), high density polyethylene (so-called HDPE), polypropylene (propylene homopolymer), polypropylene random copolymer, poly 1-butene, poly 4-methyl-1-pentene, ethylene·propylene random copolymer, ethylene·1-butene random copolymer and propylene·1-butene random copolymer; and mixtures of the thermoplastic resins.

Among these thermoplastic resins (B), from the viewpoint of spinning stability during molding and drawing processability of the nonwoven fabric, high-pressure low density polyethylene, linear low density polyethylene (so-called LLDPE), high density polyethylene, and propylene polymers such as polypropylene and a polypropylene random copolymer are further preferable.

The propylene polymer is preferably a propylene homopolymer or a copolymer of propylene and a very small amount of one or two or more $\alpha$-olefinshaving 2 or more carbon atoms (excluding 3 carbon atoms), and preferably 2 to 8 carbon atoms (excluding 3 carbon atoms) such as ethylene, 1-butene, 1-pentene, 1-hexene, 1-octene, and 4-methyl-1-pentene, having a melting point (Tm) of 155°C or higher, and preferably 157°C to 165°C.

The melt flow rate (MFR: ASTM D-1238, 230°C, load: 2160 g) of the propylene polymer is not particularly limited as long as the propylene polymer can be melt-spun. The melt flow rate of the propylene polymer is usually 1 g/10 min to 1000 g/10 min, more preferably 5 g/10 min to 500 g/10 min, and more preferably 10 g/10 min to 100 g/10 min. The ratio Mw/Mn of the weight average molecular weight (Mw) of the propylene polymer to the number average molecular weight (Mn) is usually 1.5 to 5.0. Mw/Mn is preferably in the range of 1.5 to 3.0 in that fibers having good spinnability and excellent fiber strength can be obtained. Mw and Mn can be measured by a known method using GPC (gel permeation chromatography).

From the viewpoint of further improving the drawing processing properties of the obtained multilayer nonwoven fabric, it is preferable that the thermoplastic resin (B) is an olefinic polymer composition obtained by adding HDPE to a propylene polymer. In this case, the ratio of HDPE is preferably 1% by mass to 20% by mass, more preferably 2% by mass to 15% by mass, and further preferably 4% by mass to 10% by mass based on 100% by mass of the total amount of the propylene polymer and HDPE.

The type of HDPE to be added to the propylene polymer is not particularly limited, but HDPE usually has a density is 0.94 g/cm$^3$ to 0.97 g/cm$^3$, preferably 0.95 g/cm$^3$ to 0.97 g/cm$^3$, and more preferably 0.96 g/cm$^3$ to 0.97 g/cm$^3$. HDPE has a melt flow rate (MFR: ASTM D-1238, 190°C, load: 2160 g), which is not particularly limited as long as it has spinnability, of usually 0.1 to 100 g/10 min, preferably 0.5 to 50 g/10 min, and more preferably 1 to 30 g/10 min from the viewpoint of exhibiting extension properties. In the disclosure, good spinnability means that filament breakage is not caused and filament fusion is not caused during discharging from a spinning nozzle and during drawing.

<Additive>

Various stabilizers such as a heat stabilizer, a weather-resistant stabilizer, and an antioxidant, an antistatic agent, a slip agent, an anti-fogging agent, a lubricant, a dye, a pigment, a natural oil, a synthetic oil, and wax and the like can be added to the elastic nonwoven fabric and the mixed-fiber spunbond nonwoven fabric if necessary.

(Other layers)

The multilayer nonwoven fabric of the disclosure may have one or more layers other than the elastic nonwoven fabric and the mixed-fiber spunbond nonwoven fabric depending on applications.

Specific examples of the other layer include a knitted fabric, a woven fabric, a nonwoven fabric other than the elastic nonwoven fabric and the mixed-fiber spunbond nonwoven fabric, a natural fiber such as cotton, and a film. A method of further stacking (adhering) another layer on the multilayer nonwoven fabric of the disclosure is not particularly limited, and various method such as a thermal fusion method such as thermal embossing or ultrasonic fusion, a mechanical confounding method such as needle punch or water jet, a method of using an adhesive such as a hot melt adhesive or a urethane adhesive, and extrusion laminating may be employed.

Examples of nonwoven fabrics other than the elastic nonwoven fabric and the mixed-fiber spunbond nonwoven fabric, included in the multilayer nonwoven fabric of the disclosure include various known nonwoven fabrics such as a spunbond nonwoven fabric, a meltblown nonwoven fabric, a wet nonwoven fabric, a dry nonwoven fabric, a dry pulp nonwoven fabric, a flash spun nonwoven fabric, and a split non-woven fabric. The nonwoven fabric may be a stretchable nonwoven fabric or a nonstretchable nonwoven fabric. Herein, the nonstretchable nonwoven fabric refers to a nonwoven fabric which does not cause recovery stress after extending in MD (machine direction of the nonwoven fabric, longitudinal direction) or CD (direction perpendicular to the machine direction of the nonwoven fabric, lateral direction).

The film included in the multilayer nonwoven fabric of the disclosure is preferably an air breathable (moisture permeable) film from the viewpoint of maintaining air breathability and hydrophilicity which are features of the multilayer nonwoven fabric of the disclosure. Examples of the air breathable film include various known air breathable films such as a film made of moisture permeable thermoplastic elastomer such as a polyurethane elastomer, a polyester elastomer, or a polyamide elastomer, and a porous film obtained by drawing a film made of a thermoplastic resin containing inorganic fine particles or organic fine particles to create pores in the film. As the thermoplastic resin used for the porous film, polyolefins such as high pressure low density polyethylene, linear low density polyethylene (so-called LLDPE), high density polyethylene, polypropylene, a polypropylene random copolymer, and combinations thereof are preferable. In a case in which it is not necessary to maintain the air breathability and hydrophilicity of the multilayer nonwoven fabric, thermoplastic resin films made of polyethylene, polypropylene, or combinations thereof and the like may be used.

(Method of manufacturing multilayer nonwoven fabric)

The multilayer nonwoven fabric of the disclosure can be manufactured by a known nonwoven-fabric manufacturing method using an α-olefin copolymer in which a ratio (E40/E23) between a storage elastic modulus E40 at 40°C and a storage elastic modulus E23 at 23°C is 37% or higher, as a raw material of the elastic nonwoven fabric, the thermoplastic elastomer (A) and the thermoplastic resin (B) as raw materials of the mixed-fiber spunbond nonwoven fabric, and an additive if necessary.

As an example of a method of producing a multilayer nonwoven fabric, a method of using a nonwoven fabric producing apparatus having at least two-line spinning devices will be described below.

First, a thermoplastic elastomer (A) and a thermoplastic resin (B) are melted by an extruder provided in a first-line spinning device, introduced to a spinneret (die) fitted with a large number of spinning holes (nozzles) or sheath-core spinning holes if necessary, and discharged. Thereafter, the long fibers composed of the melt-spun thermoplastic elastomer (A) and the long fibers composed of the thermoplastic resin (B) are introduced into a cooling chamber, and cooled by cooling air. The long fibers are then drawn (towed) by drawing air, and the mixed-fiber spunbond nonwoven fabric is deposited on a moving collection surface.

On the other hand, the α-olefin copolymer in which a ratio (E40/E23) between a storage elastic modulus E40 at 40°C and a storage elastic modulus E23 at 23°C is 37% or higher is melted by an extruder provided in a second-line spinning device. The melted α-olefin copolymer in which a ratio (E40/E23) between a storage elastic modulus E40 at 40°C and a storage elastic modulus E23 at 23°C is 37% or higher is introduced into a spinning hole with a spinneret including many spinning holes (nozzles), and is then discharged. Thereafter, a melt-spun long fiber of the α-olefin copolymer in which a ratio (E40/E23) between a storage elastic modulus E40 at 40°C and a storage elastic modulus E23 at 23°C is 37% or higher is introduced into a cooling chamber, and cooled by cooling air. Thereafter, the long fibers are drawn (towed) by drawing air, and deposited on the mixed-fiber spunbond nonwoven fabric, thereby forming an elastic nonwoven fabric.

If necessary, a mixed-fiber spunbond nonwoven fabric may be deposited on the elastic nonwoven fabric by using a third-line spinning device.

The melting temperatures of polymers serving as raw materials for the elastic nonwoven fabric and the mixed-fiber spunbond nonwoven fabric are not particularly limited as long as the temperatures are equal to or higher than the softening temperature or melting temperature of each polymer and lower than the thermal decomposition temperature. The temperature of the spinneret depends on the type of the polymer to be used. For example, in a case in which a thermoplastic polyurethane elastomer or an olefin copolymer elastomer is used as the thermoplastic elastomer (A), and a propylene polymer or an olefin polymer composition of a propylene polymer and HDPE is used as the thermoplastic resin (B), the temperature of the spinneret is usually set to a temperature of from 180°C to 240°C, preferably from 190 to 230°C, and more preferably from 200 to 225°C.

The temperature of the cooling air is not particularly limited as long as the polymer can be solidified, but it is usually from 5°C to 50°C, preferably from 10°C to 40°C, and more preferably from 15°C to 30°C. A drawing air velocity is usually in the range of from 100 m/min to 10,000 m/min, and preferably from 500 m/min to 10,000 m/min.

The multilayer nonwoven fabric of the disclosure preferably has a structure in which at least a part of the elastic nonwoven fabric and at least a part of the mixed-fiber spunbond nonwoven fabric are thermally fusion bonded. At this time, at least a part of the elastic nonwoven fabric and at least a part of the mixed-fiber spunbond nonwoven fabric may be compacted with nip rolls before thermal fusion bonding.

The method of thermal fusion bonding is not particularly limited, and it can be selected from various known methods. Examples of prebonding methods include a method of using means such as ultrasonic waves, thermal embossing using embossing rolls, and a method using hot air through. Among these, from the viewpoint that the long fibers are efficiently drawn during drawing, thermal embossing is preferable, and the temperature range thereof is preferably from 60°C to 115°C.

In a case in which a part of the multilayer is thermally fusion bonded by thermal embossing, the emboss area ratio is usually from 5% to 30%, and preferably from 5% to 20%, and the non-embossed unit area is 0.5 mm$^2$ or more, and preferably from 4 mm$^2$ to 40 mm$^2$. The non-embossed unit area is the maximum area of a quadrangle inscribed in emboss in a non-embossed portion of minimum units surrounded by embossed portions in all directions. Examples of the shape of the mark include a circle, an ellipse, an oval, a square, a rhombus, a rectangle, a square, and continuous shapes based on these shapes.

<Stretchable multilayer nonwoven fabric>

The stretchable multilayer nonwoven fabric of the disclosure is a multilayer nonwoven fabric having stretchability obtained by drawing the multilayer nonwoven fabric.

The stretchable multilayer nonwoven fabric of the disclosure can be obtained by drawing the multilayer nonwoven fabric. The drawing method is not particularly limited, and conventionally known methods can be applied. The drawing method may be such that the multilayer nonwoven fabric is drawn partially or entirely. The drawing method may be such that the multilayer nonwoven fabric is drawn uniaxially or biaxially. Examples of the method of drawing the multilayer nonwoven fabric in a machine direction (MD) include a method of passing the partially fusion bonded mixed fibers through two or more pairs of nip rolls. At this time, the partially fusion bonded multilayer nonwoven fabric can be drawn by increasing the rotational speeds of the nip rolls in order in the machine direction. Gear drawing can also be performed using a gear drawing device shown in Fig. 1.

The draw ratio is preferably 50% or more, more preferably 100% or more, and further preferably 200% or more, and preferably 1000% or less, and more preferably 400% or less.

In the case of uniaxial drawing, the above draw ratio is preferably satisfied in the machine direction (MD) or a direction perpendicular thereto (CD). In the case of biaxial drawing, the above draw ratio is preferably satisfied in at least one of the machine direction (MD) or the direction perpendicular thereto (CD).

By drawing at the above draw ratio, all the (long) fibers forming the elastic nonwoven fabric and the mixed-fiber spunbond nonwoven fabric are drawn. The long fibers forming the mixed-fiber spunbond nonwoven fabric layer are plastically deformed to be extended (that is, lengthened) in accordance with the above draw ratio.

Therefore, in a case in which the stress is released after the multilayer nonwoven fabric is drawn, the (long) fibers forming the elastic nonwoven fabric are elastically recovered, and the long fibers forming the mixed-fiber spunbond nonwoven fabric are folded without being elastically recovered. As a result, the multilayer nonwoven fabric exhibits bulkiness. In addition, since the long fibers forming the mixed-fiber spunbond nonwoven fabric tend to become thinner, it is considered that the long fibers can have improved flexibility and tactile feeling, as well as an extension stop function.

<Fiber product>

The fiber product of the disclosure includes the multilayer nonwoven fabric or the stretchable multilayer nonwoven fabric of the disclosure. The fiber product is not particularly limited, and examples thereof include absorbent articles such as disposable diapers and sanitary articles, hygiene articles such as sanitary masks, medical articles such as bandages, clothing materials, and packaging materials. The fiber product of the disclosure preferably includes the multilayer nonwoven fabric or the stretchable multilayer nonwoven fabric of the disclosure as an elastic member.

[Examples]

Below, the invention will be more specifically described using examples. However, the invention is not limited to those examples. Materials, doses, percentages, procedures, and the like described in the following examples can be appropriately modified within a scope not departing from the essence of the disclosure. Property values and the like in the examples and a comparative example were measured and evaluated by the following method.

(1) Basis weight [g/m$^2$]

Six test pieces of 200 mm (machine direction: MD) $\times$ 50 mm (lateral direction: CD) were sampled from a nonwoven fabric simple body or a multilayer nonwoven fabric. Three sampling points were arbitrarily selected for both MD and CD (6 points in total). Subsequently, the mass (g) of each of the sampled test pieces was measured using a scale electric balance (manufactured by Kensei Co., Ltd.). The average value of the masses of the test pieces was determined. The determined average value was converted to the mass (g) per 1 m$^2$ to the first decimal place to give a basis weight [g/m$^2$] of each sample.

(2) Spinnability

During manufacture of a nonwoven fabric in each example, a spinning state near a nozzle surface in an apparatus of manufacturing a spunbond nonwoven fabric was visually observed, and the number of yarn breakages per five minutes (unit: the number of times/ 5 min.) was counted. When the number of yarn breakages is 0/5 min., the spinnability was evaluated as "A". When no nonwoven fabric was obtained due to occurrence of yarn breakage, the spinnability was evaluated as "C" (Table 1).

(3) Extrudability

An extruder having 75 mm$\phi$ was fed with 20 kg of raw material, and the extrusion stability was checked for 30 minutes.
A: stable extrusion for 30 minutes.
C: occurrence of blocking of the raw material at the base of the extruder during a 30-minute period.

(4) Maximum Load [N/50 mm] and Maximum-Load Elongation [%]

Five test samples each of 50 mm (MD) $\times$ 200 mm (CD) were taken from a multilayer nonwoven fabric. Spots where the test samples were taken are freely-selected five spots. Then, each of the taken test samples was subjected to a tensile test under the conditions of a chuck-to-chuck distance of 100 mm and a tensile speed of 100 mm/min., using a universal tensile tester (manufactured by INTESCO co., ltd., IM-201 Type), thereby obtaining a load (maximum load [N/50 mm]) and an elongation (maximum-load elongation [%]) at a maximum load point. Additionally, each average of maximum loads and maximum-load elongations at the above-described five spots was obtained, and the obtained average was rounded off to the first decimal place.

(5) Residual Strain [%], 50% Elongation Stress [N/50 mm], 50% Recovery Stress [N/50 mm], and Stretch Property

Five test samples each of 50 mm (MD) $\times$ 200 mm (CD) were taken from a multilayer nonwoven fabric. Spots where the test samples were taken are freely-selected five spots. Then, each of the taken test samples was drawn under the conditions of a chuck-to-chuck distance of 100 mm, a tensile speed of 100 mm/min., and a draw ratio of 100%, using a universal tensile tester (manufactured by INTESCO co., ltd., IM-201 Type), and was immediately caused to recover its original length at the same speed. Two cycles of this operation were performed. Then, an elongation at the time when a stress started to rise in the second cycle was measured, and the measured stress was defined as a residual stress [%]. Subsequently, a stress at the time when the draw ratio became equal to 50% during elongation in the second cycle was defined as a 50% elongation stress, and a stress at the time when the draw ratio became equal to 50% during recovery in the second cycle was defined as a 50% recovery stress. Further, a value of [50% elongation stress $\div$ 50% recovery stress] in the second cycle was measured, and the measured value was defined as a measure of a stretch property. As each value of a residual strain and a value of [50% elongation stress $\div$ 50% recovery stress] becomes smaller, it means that a stretch property is more excellent. Additionally, each average of residual strains and stretch properties at the above-described five spots was obtained, and the obtained average was rounded off to the second decimal place. When a stretch property was 3.0 or smaller, the stretch property was evaluated as "A", and when a stretch property was larger than 3.0, the stretch property was evaluated as "C".

Besides, regarding a storage elastic modulus at each temperature and a ratio between storage elastic moduli in each example, results from measurement by the above-described measuring method were shown in Table 1. Note that when a ratio (E40/E23) between a storage elastic modulus E40 at 40°C and a storage elastic modulus E23 at 23°C was 37% or higher, the stress maintainability was evaluated as "A", and when the ratio was lower than 37%, the stress maintainability was evaluated as "C".

[Example 1]

<Preparation of Thermoplastic Polyurethane Elastomer (TPU)>

71.7 parts by mass of a polyester polyol having a number average molecular weight of 1932, 4.8 parts by mass of 1,4-butanediol (hereinafter abbreviated as "BD"), 0.3 parts by mass of pentaerythritol tetrakis[3-(3,5-di-t-butyl-4-hydroxyphenyl)propionate (hereinafter abbreviated as "antioxidant-1"), and 0.3 parts by mass of polycarbodiimide were mixed to obtain a mixture, and 22.9 parts by mass of MDI was added to the mixture, followed by sufficiently performing high speed stirring and mixing. Thereafter, the reaction was carried out at 160°C for 1 hour to obtain a reaction product. After the reaction product was pulverized, 0.8 parts by mass of ethylenebisstearic acid amide, 0.5 parts by mass of triethylene glycol-bis-[3-(3,5-di-t-butyl-4-hydroxyphenyl)propionate] (hereinafter abbreviated as "antioxidant-2"), and 0.8 part by mass of ethylene bisoleic acid amide (hereinafter abbreviated as "EOA") were mixed with 100 parts by mass of the pulverized product to obtain a mixture. Thereafter, the mixture was melt-kneaded in an extruder (set temperature: 210°C) and granulated to obtain a thermoplastic polyurethane elastomer [TPU (A-1)].

The physical properties of the obtained TPU (A-1) were hardness: 81, melt viscosity: 1.1, and flow starting temperature: 155°C.

<Preparation of Thermoplastic Resin Composition for Mixed-fiber spunbond nonwoven Fabric>

92 parts by mass of a propylene homopolymer (hereinafter abbreviated as "PP-1") having a MFR (measured at 230°C under a load of 2.16 kg in accordance with ASTM D 1238) of 60 g/10 min, a density 0.91 g/cm$^3$, and a melting point of 160°C, and 8 parts by mass of a high density polyethylene (hereinafter abbreviated as "HDPE") having MFR (measured at 190°C under a load of 2.16 kg in accordance with ASTM D 1238) of 5 g/10 min, a density of 0.97 g/cm$^3$ and a melting point of 134°C were mixed to prepare a thermoplastic resin composition (B-1).

<$\alpha$-Olefin copolymer for elastic nonwoven fabric>

As an elastic nonwoven fabric, an ethylene-propylene copolymer with a trade name "VISTAMAXX ™6202" manufactured by Exxon Mobile Chemical Ltd. was used. The copolymer had an MFR (at 230°C and at a load of 2.16 kg) of 20 g/10 min., an ethylene content of 15% by mass, a tensile elastic modulus of 9.8 MPa, a storage elastic modulus at 23°C of 12.0 MPa, a storage elastic modulus at 40°C of 6.55 MPa, a melting point of 110.4°C, and a fusion heat amount of 5.89 mJ/mg, and a glass transition temperature of -32.6°C.

<Manufacture of multilayer nonwoven fabric>

The TPU (A-1) and the thermoplastic resin composition (B-1) prepared in the above-described manner were melted independently of each other, using an extruder having 50 mm$\phi$ and an extruder having 75 mm$\phi$, respectively. Thereafter, the materials were melt-spun by a spunbond method under the conditions of a resin temperature and a die temperature each of 205°C, a cooling-air temperature of 20°C, and a drawing-air speed of 3200 m/min., using a spunbond-nonwoven-fabric molding apparatus (having a length of 800 mm along a direction perpendicular to a machine direction on a collecting surface) including a spinneret. Then, a web including mixed long fibers including a long fiber A formed of the TPU (A-1) and a long fiber B formed of the thermoplastic resin composition (B-1) was deposited on the collecting surface.

More specifically, a nozzle pattern in which discharge holes for the TPU (A-1) and discharge holes for the B-1 were in a staggered arrangement was used as the spinneret, the nozzle diameter for the TPU (A-1) (long fiber A) was 0.75 mm$\phi$, and the nozzle diameter for the B-1 (long fiber B) was 0.6 mm$\phi$. A vertical nozzle pitch was 8 mm and a horizontal nozzle pitch was 11 mm. A ratio between the numbers of nozzles was set such that nozzles for the long fiber A and nozzles for the long fiber B were in a ratio of 1:1.45. A single-hole discharge amount of the long fiber A was 0.82 g/(min. hole), a single-hole discharge amount of the long fiber B was 0.56 g/(min. hole). Then, a spunbond nonwoven fabric including mixed long fibers was deposited on the collecting surface, as a first layer.

Subsequently, an elastic nonwoven fabric was deposited on the mixed-fiber spunbond nonwoven fabric, as a second layer. More specifically, the above-described VISTAMAXX 6202 was melted using a uniaxial extruder having a screw diameter of 75 mm$\phi$. Thereafter, the material was melt-spun by a spunbond method under the conditions of a resin temperature and a die temperature each of 290°C, a cooling-air temperature of 20°C, and a drawing-air speed of 2888 m/min., using a spunbond-nonwoven-fabric molding apparatus (having a length of 800 mm along a direction perpendicular to a machine direction on a collecting surface) including a spinneret, thereby depositing the molded fabric as the second layer.

Subsequently, a mixed-fiber spunbond nonwoven fabric similar to the first layer was deposited on the elastic nonwoven fabric, as a third layer, in the same manner as the first layer, thereby producing a three-layered deposit. This deposit was subjected to a heating and pressing treatment (an embossed-area percentage of 18%, an embossing temperature of 80°C) by an embossing roll, thereby producing a multilayer nonwoven fabric in which the total basis weight was 65.0 g/m$^2$, each basis weight of the first and third layers was 20.0 g/m$^2$, and the basis weight of the second layer was 25.0 g/m$^2$.

The extrudability of VISTAMAXX 6202 was excellent. Results from measurement of respective properties of the obtained multilayer nonwoven fabric were shown in Table 1.

[Example 2]

A multilayer nonwoven fabric was produced in the same manner as in the first example except that an ethylene-propylene copolymer with a trade name "VISTAMAXX ™ 7050FL" manufactured by Exxon Mobile Chemical Ltd., was used as an elastic nonwoven fabric, each of a resin temperature and a die temperature of VISTAMAXX 7050FL was changed to 215°C, and an embossing temperature was changed to 90°C.

VISTAMAXX 7050FL had an MFR (at 230°C and at a load of 2.16 kg) of 48 g/10 min., an ethylene content of 13% by mass, a tensile elastic modulus of 14.4 MPa, a storage elastic modulus at 23°C of 17.4 MPa, a storage elastic modulus at 40°C of 8.77 MPa, a melting point of 60.9°C, and a fusion heat amount of 19 mJ/mg, and a glass transition temperature of -30.3°C.

The extrudability of VISTAMAXX 7505FL was excellent. Results from measurement of respective properties of the obtained multilayer nonwoven fabric were shown in Table 1.

[Comparative Example 1]

A multilayer nonwoven fabric was produced in the same manner as in the first example except that the following low crystalline polypropylene was used as an elastic nonwoven fabric, each of a resin temperature and a die temperature of the low crystalline propylene was changed to 215°C, an embossing temperature was changed to 90°C, and the basis weight of the second layer was changed to 20.0 g/m$^2$ (the total basis weight was 60.0 g/m2).

<Preparation of low crystalline polypropylene for elastic nonwoven fabric>

To a stainless steel reactor having an inner volume of 0.2 m$^3$ equipped with a stirrer, n-heptane at 20 L/h, triisobutylaluminum at 15 mmol/h, and a catalyst component obtained by preliminarily contacting dimethylanilinium tetrakispentafluorophenylborate, (1,2'-dimethylsilylene)(2,1'-dimethylsilylene)-bis(3-trimethylsilylmethylindenyl)zirconium dichloride, triisobutylaluminum, and propylene at 6 µmol/h per zirconium were continuously supplied.

Propylene and hydrogen were continuously supplied in a state where a hydrogen concentration in a gas phase portion was 8 mol% at a polymerization temperature of 70°C and the total pressure in the reactor was maintained at 0.7 MPa G

To the obtained polymerization solution, 1000 ppm of SUMILIZER GP (manufactured by Sumitomo Chemical Co., Ltd.) was added, and the solvent was removed to obtain a propylene polymer.

The propylene polymer thus obtained had a mass average molecular weight (Mw) of $1.2 \times 10^4$ and Mw/Mn of 2. According to NMR measurement, [mmmm] was 46 mol%; [rrrr]/(1 - [mmmm]) was 0.038; [rmrm] was 2.7 mol%; and [mm] $\times$ [rr]/[mr]$^2$ was 1.5, and the tensile elastic modulus was 32.9 MPa.

The low crystalline polypropylene had a storage elastic modulus at 23°C of 49.3 MPa, a storage elastic modulus at 40°C of 17.8 MPa, a melting point of 56.1°C, and a fusion heat amount of 30.0 mJ/mg, and a glass transition temperature of -7.3°C.

As blocking occurred, the extrudability of the low crystalline polypropylene was poor. Results from measurement of respective properties of the obtained multilayer nonwoven fabric were shown in Table 1.

As appreciated from comparison with the comparative example 1, the example 1 and the example 2, in which a stretch property (50% elongation stress/50% recovery stress) was reduced, improved stretchability. It is further appreciated that in the example 1 and the example 2, a ratio (E40/E23) between a storage elastic modulus E40 at 40°C and a storage elastic modulus E23 at 23°C is high, and reduction of elasticity of the elastic nonwoven fabric in a temperature changing environment (from 40°C to 23°C) can be more easily suppressed than that in the comparative example 1. Thus, the multilayer nonwoven fabric according to the disclosure is a multilayer nonwoven fabric excellent in a stretch property and excellent in stress maintainability.

[Table 1]

| | | | Example 1 | | Example 2 | | Comparative Example 1 | |
|---|---|---|---|---|---|---|---|---|
| Spunbond nonwoven fabric (a) | Resin | - | Thermoplastic elastomer (A) | Thermoplastic resin (B) | Thermoplastic elastomer (A) | Thermoplastic resin (B) | Thermoplastic elastomer (A) | Thermoplastic resin (B) |
| | | | TPU | pp-1/HDPE | TPU | pp-1/HDPE | TPU | pp-1/HDPE |
| | Percentage | [%] | 55 | 45 | 55 | 45 | 55 | 45 |
| | Basis weight | [g/m$^2$] | 20 | | 20 | | 20 | |
| | Spinnability | - | A | | A | | A | |
| Elastic nonwoven fabric (b) | Resin | - | VISTAMAXX 6202 | | VISTAMAXX 7050FL | | Low crystalline PP | |
| | Composition | - | C2/C3 | | C2/C3 | | C3 | |
| | C2 percentage | [%] | 15 | | 13 | | 0 | |
| | Glass transition temperature | [°C] | -32.6 | | -30.3 | | -7.3 | |
| | Melting point | [°C] | 110.4 | | 60.9 | | 56.1 | |
| | Fusion heat amount | [mJ/mg] | 5.89 | | 19 | | 30.0 | |
| | Storage elastic modulus — 23°C | [MPa] | 12 | | 17.4 | | 49.3 | |
| | Storage elastic modulus — 40°C | [MPa] | 6.55 | | 8.77 | | 17.8 | |
| | Storage elastic modulus — 40°C/23°C | [%] | 54.6 | | 50.4 | | 35.9 | |
| | Basis weight | [g/m$^2$] | 25 | | 25 | | 25 | |
| | Extrudability | - | A | | A | | C | |
| | Spinnability | - | A | | A | | A | |

(continued)

| | | | Example 1 | Example 2 | Comparative Example 1 |
|---|---|---|---|---|---|
| Multilayer nonwoven fabric (a/b/a) | Total basis weight | [g/m²] | 65 | 65 | 65 |
| | Maximum load | [N/50 mm] | 16.4 | 141 | 37.1 |
| | Maximum-load elongation | [%] | 264 | 236 | 290 |
| | Residual strain | [%] | 12.3 | 18.8 | 21.0 |
| | 50% elongation stress (E) | [N/50 mm] | 0.91 | 0.93 | 1.28 |
| | 50% recovery stress (R) | [N/50 mm] | 0.46 | 0.36 | 0.39 |
| | Stretch property (E/R) | - | 1.98 | 2.58 | 3.28 |
| | Stretch property (evaluation) | - | A | A | C |
| | Stress Maintainability | - | A | A | C |

**EP 3 779 016 B1**

**Claims**

1. A multilayer nonwoven fabric, comprising:

an elastic nonwoven fabric containing an $\alpha$-olefin copolymer in which a ratio (E40/E23) between a storage elastic modulus E40 at 40°C and a storage elastic modulus E23 at 23°C is 37% or higher; and a mixed-fiber spunbond nonwoven fabric that is disposed on at least one side of the elastic nonwoven fabric, wherein the $\alpha$-olefin copolymer includes a copolymer of ethylene and propylene, and the mixed-fiber spunbond nonwoven fabric contains a long fiber of a thermoplastic elastomer (A) and a long fiber of a thermoplastic resin (B), which is different from the thermoplastic elastomer (A) and which is a polyolefin, in a ratio (A) : (B) of from 10 to 90% by mass : from 90 to 10% by mass, wherein (A) + (B) = 100% by mass.

2. The multilayer nonwoven fabric according to claim 1, wherein the storage elastic modulus E23 at 23°C of the $\alpha$-olefin copolymer is 45 MPa or lower.

3. The multilayer nonwoven fabric according to claim 1 or 2, wherein the $\alpha$-olefin copolymer has a tensile elastic modulus of 30 MPa or lower.

4. The multilayer nonwoven fabric according to any one of claims 1 to 3, wherein a maximum point elongation of the long fiber of the thermoplastic resin (B), in a case of being formed into the spunbond nonwoven fabric, is 50% or more.

5. The multilayer nonwoven fabric according to any one of claims 1 to 4, wherein the thermoplastic elastomer (A) is a thermoplastic polyurethane elastomer.

6. The multilayer nonwoven fabric according to claim 5, wherein the thermoplastic polyurethane elastomer is a thermoplastic polyurethane elastomer satisfying the following relational expression (I):

$$a/(a + b) \leq 0.8 \,(\mathrm{I})$$

wherein:

a represents a total amount of fusion heat calculated from an endothermic peak in a range of 90°C to 140°C, as measured by DSC; and b represents a total amount of fusion heat calculated from an endothermic peak in a range from higher than 140°C to 220°C or lower, as measured by DSC.

7. The multilayer nonwoven fabric according to any one of claims 1 to 6, wherein the thermoplastic resin (B) is a propylene copolymer.

8. The multilayer nonwoven fabric according to any one of claims 1 to 7, wherein the thermoplastic resin (B) includes 99 to 80% by mass of a propylene polymer and 1 to 20% by mass of a high-density polyethylene.

9. A stretchable multilayer nonwoven fabric obtained by drawing the multilayer nonwoven fabric according to any one of claims 1 to 8.

10. A fiber product including the multilayer nonwoven fabric according to any one of claims 1 to 8 or the stretchable multilayer nonwoven fabric according to claim 9.

11. An absorbent article comprising the multilayer nonwoven fabric according to any one of claims 1 to 8 or the stretchable multilayer nonwoven fabric according to claim 9.

12. A sanitary mask comprising the multilayer nonwoven fabric according to any one of claims 1 to 8 or the stretchable multilayer nonwoven fabric according to claim 9.

**Patentansprüche**

1. Mehrlagiger Vliesstoff, umfassend:

   einen elastischen Vliesstoff, enthaltend ein α-Olefin-Copolymer, bei dem ein Verhältnis (E40/E23) zwischen einem Speicherelastizitätsmodul E40 bei 40°C und einem Speicherelastizitätsmodul E23 bei 23°C 37% oder mehr beträgt; und
   einen Mischfaser-Spinnvliesstoff, der auf mindestens einer Seite des elastischen Vliesstoffs angeordnet ist, wobei das α-Olefin-Copolymer ein Copolymer von Ethylen und Propylen enthält, und
   der Mischfaser-Spinnvliesstoff eine Langfaser eines thermoplastischen Elastomers (A) und eine Langfaser eines thermoplastischen Harzes (B), das sich von dem thermoplastischen Elastomer (A) unterscheidet und das ein Polyolefin ist, in einem Verhältnis (A) : (B) von 10 bis 90 Massen-% : 90 bis 10 Massen-% enthält, wobei (A) + (B) = 100 Massen-%.

2. Mehrlagiger Vliesstoff gemäß Anspruch 1, wobei der Speicherelastizitätsmodul E23 bei 23°C des α-Olefin-Copolymers 45 MPa oder weniger beträgt.

3. Mehrlagiger Vliesstoff gemäß Anspruch 1 oder 2, wobei das α-Olefin-Copolymer einen Zugelastizitätsmodul von 30 MPa oder weniger aufweist.

4. Mehrlagiger Vliesstoff gemäß einem der Ansprüche 1 bis 3, wobei die maximale Punktdehnung der Langfaser des thermoplastischen Harzes (B) im Falle des Formens des Spinnvlieses 50% oder mehr beträgt.

5. Mehrlagiger Vliesstoff gemäß einem der Ansprüche 1 bis 4, wobei das thermoplastische Elastomer (A) ein thermoplastisches Polyurethan-Elastomer ist.

6. Mehrlagiger Vliesstoff gemäß Anspruch 5, wobei das thermoplastische Polyurethan-Elastomer ein thermoplastisches Polyurethan-Elastomer ist, das den folgenden Beziehungsausdruck (I) erfüllt:

$$a/(a + b) \leq 0,8 \quad (I)$$

   worin:

   a für eine Gesamtmenge der Schmelzwärme steht, die aus einem endothermen Peak im Bereich von 90°C bis 140°C, gemessen durch DSC, berechnet wird; und
   b für eine Gesamtmenge der Schmelzwärme steht, die aus einem endothermen Peak im Bereich von mehr als 140°C bis 220°C oder weniger, gemessen durch DSC, berechnet wird.

7. Mehrlagiger Vliesstoff gemäß einem der Ansprüche 1 bis 6, wobei das thermoplastische Harz (B) ein Propylen-Copolymer ist.

8. Mehrlagiger Vliesstoff gemäß einem der Ansprüche 1 bis 7, wobei das thermoplastische Harz (B) 99 bis 80 Massen-% eines Propylen-Polymers und 1 bis 20 Massen-% eines Polyethylens hoher Dichte enthält.

9. Dehnbarer mehrlagiger Vliesstoff, erhalten durch Ziehen des mehrlagigen Vliesstoffs gemäß einem der Ansprüche 1 bis 8.

10. Faserprodukt, enthaltend den mehrlagigen Vliesstoff gemäß einem der Ansprüche 1 bis 8 oder den dehnbaren mehrlagigen Vliesstoff gemäß Anspruch 9.

11. Saugfähiger Artikel, umfassend den mehrlagigen Vliesstoff gemäß einem der Ansprüche 1 bis 8 oder den dehnbaren mehrlagigen Vliesstoff gemäß Anspruch 9.

12. Hygienemaske, umfassend den mehrlagigen Vliesstoff gemäß einem der Ansprüche 1 bis 8 oder den dehnbaren mehrlagigen Vliesstoff gemäß Anspruch 9.

**EP 3 779 016 B1**

**Revendications**

1. Étoffe non tissée multicouche, comprenant :

   une étoffe non tissée élastique contenant un copolymère d'α-oléfine dans lequel un rapport (E40/E23) entre un module d'élasticité de stockage E40 à 40 °C et un module d'élasticité de stockage E23 à 23 °C est de 37 % ou supérieur ; et
   une étoffe non tissée filée-liée à fibres mélangées qui est disposée sur au moins un côté de l'étoffe non tissée élastique,
   dans laquelle le copolymère d'α-oléfine comporte un copolymère d'éthylène et de propylène, et
   l'étoffe non tissée filée-liée à fibres mélangées contient une fibre longue d'un élastomère thermoplastique (A) et une fibre longue d'une résine thermoplastique (B), qui est différente de l'élastomère thermoplastique (A) et qui est une polyoléfine, en un rapport (A) : (B) de 10 à 90 % en masse : de 90 à 10 % en masse, dans lequel (A) + (B) = 100 % en masse.

2. Étoffe non tissée multicouche selon la revendication 1, dans laquelle le module d'élasticité de stockage E23 à 23 °C du copolymère d'α-oléfine est de 45 MPa ou inférieur.

3. Étoffe non tissée multicouche selon la revendication 1 ou 2, dans laquelle le copolymère d'α-oléfine a un module d'élasticité en traction de 30 MPa ou inférieur.

4. Étoffe non tissée multicouche selon l'une quelconque des revendications 1 à 3, dans laquelle un allongement ponctuel maximal de la fibre longue de la résine thermoplastique (B), dans un cas où elle mise sous la forme de l'étoffe non tissée filée-liée, est de 50 % ou plus.

5. Étoffe non tissée multicouche selon l'une quelconque des revendications 1 à 4, dans laquelle l'élastomère thermoplastique (A) est un élastomère de polyuréthane thermoplastique.

6. Étoffe non tissée multicouche selon la revendication 5, dans laquelle l'élastomère de polyuréthane thermoplastique est un élastomère de polyuréthane thermoplastique satisfaisant l'expression relationnelle (I) suivante :

$$a/(a + b) \leq 0,8 \ (I)$$

   dans laquelle :

   a représente une quantité totale de chaleur de fusion calculée à partir d'un pic endothermique dans une gamme allant de 90 °C à 140 °C, telle que mesurée par ACD ; et
   b représente une quantité totale de chaleur de fusion calculée à partir d'un pic endothermique dans une gamme allant de plus de 140 °C à 220 °C ou moins, telle que mesurée par ACD.

7. Étoffe non tissée multicouche selon l'une quelconque des revendications 1 à 6, dans laquelle la résine thermoplastique (B) est un copolymère de propylène.

8. Étoffe non tissée multicouche selon l'une quelconque des revendications 1 à 7, dans laquelle la résine thermoplastique (B) comporte de 99 à 80 % en masse d'un polymère de propylène et de 1 à 20 % en masse d'un polyéthylène à haute densité.

9. Étoffe non tissée multicouche étirable obtenue par le fait d'étirer l'étoffe non tissée multicouche selon l'une quelconque des revendications 1 à 8.

10. Produit fibreux comportant l'étoffe non tissée multicouche selon l'une quelconque des revendications 1 à 8 ou l'étoffe non tissée multicouche étirable selon la revendication 9.

11. Article absorbant comprenant l'étoffe non tissée multicouche selon l'une quelconque des revendications 1 à 8 ou l'étoffe non tissée multicouche étirable selon la revendication 9.

12. Masque sanitaire comprenant l'étoffe non tissée multicouche selon l'une quelconque des revendications 1 à 8 ou l'étoffe non tissée multicouche étirable selon la revendication 9.

FIG.1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H07503502 A
- JP 2009062667 A
- JP 2009079341 A
- WO 2016143833 A
- WO 2007138733 A1
- WO 2016143833 A1
- WO 2009111185 A2
- JP 2004244791 A